(19) Europäisches Patentamt
European Patent Office
Office européen des brevets

(11) **EP 3 928 706 A1**

(12) **EUROPEAN PATENT APPLICATION**
published in accordance with Art. 153(4) EPC

(43) Date of publication:
**29.12.2021 Bulletin 2021/52**

(21) Application number: **19915706.6**

(22) Date of filing: **16.08.2019**

(51) Int Cl.:
*A61B 6/03* (2006.01)          *G06T 7/00* (2017.01)

(86) International application number:
**PCT/CN2019/101159**

(87) International publication number:
**WO 2020/168697 (27.08.2020 Gazette 2020/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.02.2019 CN 201910132114**

(71) Applicant: **VR Doctor Medical Technology (Shenzhen) Co., Ltd.**
**Shenzhen, Guangdong, 518035 (CN)**

(72) Inventors:
• **LEE, David Wei**
  **Shenzhen, Guangdong 518035 (CN)**
• **LEE, Stewart Ping**
  **Shenzhen, Guangdong 518035 (CN)**

(74) Representative: **Dargiewicz, Joanna**
**JD&P Patent Attorneys**
**Joanna Dargiewicz & Partners**
**Ul. Mysliborska 93A/50**
**03-185 Warszawa (PL)**

(54) **AI IDENTIFICATION METHOD OF EMBOLISM BASED ON VRDS 4D MEDICAL IMAGE, AND PRODUCT**

(57)    A method and a product for AI recognizing of embolism based on VRDS 4D medical image, the method is applied to a medical imaging apparatus, and the method includes the following steps: determining a bitmap (BMP) data source according to a plurality of scanned images of a target site of a target user, wherein the target site includes an embolism formed on a wall of a target blood vessel; generating target medical image data according to the BMP data source; performing 4D medical imaging according to the target medical image data and determining a feature attribute of the embolism according to an imaging result, wherein the feature attribute includes at least one of the following: density, crawling direction, correspondence with a site of cancer focus and edge characteristics; and determining a type of the embolism according to the features and outputting the type.

Determining, by the medical imaging apparatus, a bitmap (BMP) data source according to a plurality of scanned images of a target site of a target user — S201

Generating, by the medical imaging apparatus, target medical image data according to the BMP data source — S202

Performing, by the medical imaging apparatus, a 4D medical imaging according to the target medical image data, and determining a feature attribute of the embolism according to the imaging results — S203

Determining, by the medical imaging apparatus, a type of the embolism according to the features and outputting the type — S204

Fig. 2

## Description

## TECHNICAL FIELD

This application relates to the field of medical imaging apparatus, and in particular, to a method and a product for AI recognizing of embolism based on VRDS 4D medical images.

## BACKGROUND

In current, doctors use technologies such as Computed Tomography (CT), Magnetic Resonance Imaging (MRI), Diffusion Tensor Imaging (DTI), Positron Emission Computed Tomography (PET) to acquire information such as the shape, position and topology of the embolism tissues, however, the embolism images formed by these two-dimensional slice data can not clearly present the spatial structure and edge characteristics of embolism, and doctors can only recognize the types of embolism by experience. With the rapid development of medical imaging technology, people have put forward new demands for the recognition of embolism.

## SUMMARY

Embodiments of this application provide a method and a product for AI recognizing of embolism based on VRDS 4D medical images, in order to improve the accuracy and efficiency of the medical imaging apparatus in recognizing the embolism.

In a first aspect, embodiments of this application provide a method for AI recognizing of embolism based on VRDS 4D medical images, which is applied to medical imaging apparatus; and the method includes:

determining a bitmap (BMP0 data source according to a plurality of scanned images of a target site of a target user, wherein the target site includes an embolism formed on a wall of a target blood vessel;

generating a target medical image data according to the BMP data source, wherein the target medical image data includes a data set of the target blood vessel and a data set the embolism; first data in the data set of the target blood vessel and second data in the data set of the embolism are independent of each other, and the data set of the target blood vessel is a transfer function result of a cubic space between a surface of the target blood vessel and a tissue structure inside the target blood vessel;

performing 4D medical imaging according to the target medical image data and determining a feature attribute of the embolism according to the imaging results, wherein the feature attribute includes at least one of the following: density, crawling direction, correspondence with a site of cancer focus and edge characteristics;

determining a type of the embolism according to the features attribute and outputting the type.

In a second aspect, embodiments of this application provide an apparatus for AI recognizing of embolism based on VRDS 4D medical images, wherein the apparatus is applied to a medical imaging apparatus; the apparatus for AI recognition of embolism based on VRDS AI 4D medical image includes a processing unit and a communication unit, wherein,
the processing unit is configured to: determine a BMP data source according to a plurality of scanned images of a target site of a target user, wherein the target site includes an embolism formed on the wall of the target blood vessel; generate target medical image data according to the BMP data source, wherein the target medical image data includes a data set of the target blood vessel and a data set the embolism; the first data in the data set of the target blood vessel and the second data in the data set of the embolism are independent of each other, and the data set of the target blood vessel is a transfer function result of a cubic space between a surface of the target blood vessel and a tissue structure inside the target blood vessel; perform 4D medical imaging according to the target medical image data and determine a feature attribute of the embolism according to an imaging result, wherein the feature attribute includes at least one of the following: density, crawling direction, correspondence with a site of cancer focus and edge characteristics; and determine a type of the embolism according to the feature attribute and output the type through the communication unit.

In a third aspect, embodiments of this application provide an medical imaging apparatus, the apparatus includes a processor, a memory, a communication interface, and one or more programs, wherein the one or more programs are stored in the memory and configured to be executed by the processor, and the programs include instructions for executing the steps in any method of the first aspect of the embodiment of this application.

In a fourth aspect, embodiments of this application provide a computer readable storage medium, wherein the computer readable storage medium stores a computer program for electronic data exchange, wherein the computer program causes a computer to execute some or all of the steps described in any methods of the first aspect of the embodiment of this application.

In a fifth aspect, embodiments of this application provide a computer program product, wherein the computer program product includes a non-transitory computer-readable storage medium in which a computer program is stored, the computer program is operable to cause the computer to execute some or all of the steps described in any methods of the first aspect of the embodiment of this application. The computer program product can be a software installation package.

It can be seen that in the embodiment of this application, the medical imaging apparatus first determines a BMP data source according to a plurality of scanned images of a target site of a target user; second, generates a target medical image data according to the BMP data source; third, performs 4D medical imaging according to the target medical image data and determines a feature attribute of the embolism according to an imaging result; and finally, determines a type of the embolism according to the features attribute and outputs the type. Wherein the target site includes an embolism formed on the wall of the target blood vessel, wherein the target medical image data includes a data set of the target blood vessel and a data set of the embolism; the first data in the data set of the target blood vessel and the second data in the data set of the embolism are independent of each other, and the data set of the target blood vessel is a transfer function result of a cubic space between a surface of the target blood vessel and a tissue structure inside the target blood vessel, wherein the feature attribute includes at least one of the following: density, crawling direction, correspondence with a site of cancer focus and edge characteristics. It can be seen that the medical imaging apparatus in this application can accurately determine the type of embolism from the imaging results of 4D medical imaging, and improve the accuracy and efficiency of embolism recognition.

## BRIEF DESCRIPTION OF THE DRAWINGS

In order to describe the technical solutions in the embodiments of this application or in the prior art more clearly, the following briefly describes the accompanying drawings required for describing the embodiments or the prior art, apparently, the accompanying drawings in the following description only show some embodiments of this application, and the ordinary skill person in the art may still derive other drawings from these accompanying drawings without creative efforts.

Fig. 1 is a schematic structural diagram of an intelligent analyzing and processing system based on VRDS AI 4D medical images provided by an embodiment of this application;

Fig. 2 is a schematic flowchart of a method for AI recognizing of embolism based on VRDS 4D medical images provided by an embodiment of this application;

Fig. 3 is a schematic structural diagram of an medical imaging apparatus provided by an embodiment of this application;

Fig. 4 is a block diagram of functional units composition of an apparatus for AI recognizing of embolism based on VRDS AI 4D medical images provided by an embodiment of this application.

## DETAILED DESCRIPTION OF PREFERRED EMBODIMENTS

In order to make the skilled person in the art better understand the solution of this application, the following clearly and completely describes the technical solutions in the embodiments of this application with reference to the accompanying drawings in the embodiments of this application, apparently, the described embodiments are only some but not all of the embodiments of this application. All other embodiments obtained by the ordinary skill person in the art based on the embodiments of this application without creative efforts shall fall within the protection scope of this application.

The terms "first", "second", etc. in the specification and claims of this application and the above drawings are used to distinguish different objects, but not to describe a specific order. Furthermore, that term "including" and "have" and any variations thereof are intended to cover non-exclusive inclusion. For example, a process, method, system, product or device including a series of steps or units is not limited to the listed steps or units, but optionally further includes steps or units not listed, or optionally further includes other steps or units inherent to these processes, methods, products or devices.

Reference to an "embodiment" herein means that a particular feature, structure or characteristic described in connection with an embodiment may be included in at least one embodiment of this application. The appearances of the phrase in various places in the specification are not necessarily all referring to the same embodiment, nor are separate or alternative embodiments mutually exclusive of other embodiments. It is explicitly and implicitly to be understood by the skilled person in the art that the described embodiment may be combined with other embodiments.

The medical imaging apparatuses related to the embodiments of this application refer to various instruments that use various different media as information carriers to reproduce the internal structure of the human body as images, and their image information has a corresponding relationship spatial and temporal distribution with the actual structure of the human body. "DICOM data" refers to the raw image file data collected by medical device and reflecting the internal structure features of human body, and can include information such as Computed Tomography (CT), Nuclear Magnetic Resonance (MRI), Diffusion Tensor Imaging (DTI), Positron Emission Computed Tomography (PET-CT), "image source" refers to Texture 2D/3D image volume data generated by parsing the raw DICOM data. "VRDS" refers to Virtual Reality Doctor system.

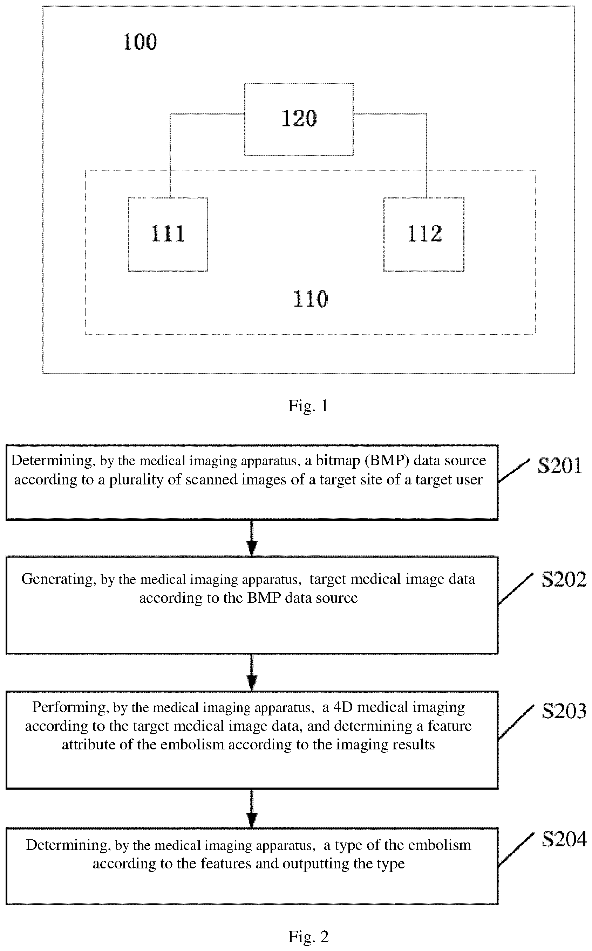

Referring to Fig. 1, Fig. 1 is a schematic structural diagram of an intelligent analyzing and processing system 100 based on VRDS AI 4D medical images provided by an embodiment of this application; the system 100 includes a medical imaging apparatus 110 and a network database 120, wherein the medical imaging apparatus 110 may include a local medical imaging apparatus 111 and/or a terminal medical imaging apparatus 112; the local medical imaging apparatus 111 or the terminal medical imaging apparatus 112 is configured to perform recognizing, positioning and four-dimensional volume drawing on the embolism of human body based on the raw DICOM data and the embolism recognition algorithm based on VRDS AI 4D medical image presented in the embodiment of this application, so as to achieve the four-dimensional stereoscopic imaging effect (the four-dimensional medical image specifically refers to the medical image including the internal spatial structure features and the external spatial structure features of the displayed tissue, the internal spatial structure features refer to slice data inside the tissues are not lost, that is, medical imaging apparatuses can present the internal constructions of tissues such as organs and blood vessels, and the external spatial structure characteristics refer to the environmental features between tissues, including spatial position characteristics (including intersection, spacing and fusion) between tissues, such as edge structure characteristics of the intersection position between kidney and artery, etc.). Compared with the terminal medical imaging apparatus 112, the local medical imaging apparatus 111 can further configured to edit the image source data to form the transfer function results of the four-dimensional human body image, which can include the transfer function results of the surface of the internal organs of the human body and the tissue structure inside the internal organs of the human body, as well as the transfer function results of the cube space, such as the number, coordinates, colors, transparency and other information of the cube editing boxes and arc editing arrays required by the transfer function. The network database 120 can be, for example, a cloud server, etc., the network database 120 is configured to store the image source generated by parsing the raw DICOM data and the transfer function result of the four-dimensional human body image edited by the local medical imaging apparatus 111, the image source can come from a plurality of local medical imaging apparatuses 111 to achieve the interactive diagnosis of a plurality of doctors.

When a user performs a specific image displaying through the medical imaging apparatus 110, the user can choose a display or a Head mounted Displays Set (HMDS) of virtual reality VR to display in combination with operation actions, which refer to the operation control of the four-dimensional human body image by the user through external intake device of the medical imaging apparatus, such as a mouse and a keyboard, so as to achieve human-computer interaction, the operation actions include at least one of the following: (1) changing the color and/or transparency of a specific organ/tissue, (2) positioning and scaling the view, (3) rotating the view to achieve multi-view 360-degree observation of the four-dimensional human image, (4) "entering" inside the organ of the human body to observe the internal construction to achieve shearing effect rendering in real time, and (5) moving the view up and down.

The following describes a method for AI recognizing of embolism based on VRDS 4D medical images in detail.

Referring to Fig. 2, Fig. 2 is a schematic flowchart of a method for AI recognizing of embolism based on VRDS 4D medical images provided by an embodiment of this application, which is applied to medical imaging apparatus as described in Fig. 1; as shown in the figure, the method for AI recognizing of embolism based on VRDS 4D medical images includes:

S201, determining, by a medical imaging apparatus, a bitmap (BMP) data source according to a plurality of scanned images associated with a target site of a target user, wherein the target site includes an embolism formed on a wall of a target blood vessel.

Wherein, the scanned image includes any one of the following: a CT image, an MRI image, a DTI image and a PET-CT image.

In this possible example, the implementation way of the medical imaging apparatus determines the BMP data source according to a plurality of scanned images of the target site of the target user can be: the medical imaging apparatus acquires a plurality of scanned images collected by a medical device and reflecting internal structure features of human body of the target user; screens at least one scanned image including the target site from the plurality of scanned images; and takes the at least one scanned image as medical digital imaging and communication DICOM data of the target user; parses the DICOM data to generate an image source of the target user; wherein the image source includes Texture 2D/3D image volume data; executes a first preset processing on the image source to obtain the BMP data source; wherein the first preset processing includes at least one of the following operations: VRDS limited contrast adaptive histogram equalization, mixed partial differential denoising and VRDS AI elastic deformation processing.

Wherein, the VRDS limited contrast adaptive histogram equalization includes: regional noise ratio limiting and global contrast limiting; the local histogram of the image source is divided into a plurality of partitions, for each partition, the slope of the transform function is determined according to the slope of the cumulative histogram of the neighborhood of the partition, and the contrast amplification degree around the pixel value of the partition is determined according to the slope of the transform function, then, according to the contrast amplification degree, the limit clipping process is carried out to generate the distribution of effective histograms and the value of effective available neighborhood size, and these clipped partial histograms are uniformly distributed to other areas of the histogram.

Wherein, the mixed partial differential denoising includes: different from Gaussian low-pass filtering (which weakens the high-frequency components of the image indiscriminately and blurs the edges of the image while denoising), the isophotes (including edges) formed by objects in the natural image should be smooth and unhindered curves, that is, the absolute value of curvature of these isophotes should be small enough, and when the image is polluted by noise, the local gray value of the image will fluctuate randomly, and it leads to the irregular oscillation of the isophote and forms the isophote with large local curvature, and according to this principle, a mixed partial differential denoising model is designed, which can protect the image edge and avoid the step effect in the smoothing process by VRDS AI curvature driving and VRDS AI high-order hybrid denoising.

Wherein, the VRDS AI elastic deformation processing includes: superimposing positive and negative random distances on the original lattice to form a difference position matrix, and then forming a new lattice at the gray level of each difference position, so as to achieve the internal distortion of the image, and further performing rotation, distortion and translation operations etc. on the image.

It can be seen that in this example, the medical imaging apparatus obtains the BMP data source by processing the raw scanned image data, which increases the information amount of the raw data and increases the depth dimension information, and finally obtains the data meeting the display requirements of the 4D medical image.

S202, generating, by the medical imaging apparatus, target medical image data according to the BMP data source, wherein the target medical image data includes a data set of the target blood vessel and a data set the embolism; first data in the data set of the target blood vessel and second data in the data set of the embolism are independent of each other; and the data set of the target blood vessel is a transfer function result of a cubic space between a surface of the target blood vessel and a tissue structure inside the target blood vessel.

In this possible example, the implementation way of the medical imaging apparatus generates the target medical image data according to the BMP data source can be: the medical imaging apparatus introduces the BMP data source into a preset VRDS medical network model to obtain first medical image data, wherein the first medical image data includes an raw data set of the target blood vessel, and the raw data set of the target blood vessel includes fusion data of the target blood vessel and the embolism; introduces the first medical image data into a preset cross blood vessel network model and performs spatial segmentation processing on the fusion data through the cross blood vessel network model to obtain a data set of the target blood vessel and a data set of the embolism; synthesizes the data set of the target blood vessel and the data set of the embolism to obtain the target medical image data.

Wherein, the cross blood vessel network model achieves the data separation of blood vessel and embolism by the following operations: (1) extracting the fusion data of the intersection position; (2) for each fusion data, separating the fusion data based on a preset data separation algorithm to obtain blood vessel boundary point data and embolism boundary point data that are independent of each other; (3) integrating multiple blood vessel boundary point data obtained after processing into first data and integrating multiple embolism boundary point data obtained after processing into second data. (4) determining the data set of the target blood vessel and the data set of the embolism according to the raw data set, the first data and the second data.

In specific implementation, the medical imaging apparatus synthesizes the data set of the target blood vessel and the data set of the embolism to obtain the target medical image data, including: the medical imaging apparatus executes a second preset processing on the data set of the target blood vessel and the data set of the embolism to obtain the target medical image data, wherein the second preset processing includes at least one of the following operations: 2D boundary optimization processing, 3D boundary optimization processing and data enhancement processing.

Wherein the 2D boundary optimization processing includes: acquiring low-resolution information and high-resolution information by sampling multiple times, wherein the low-resolution information can provide context semantic information of a segmentation target throughout the image, that is, features reflecting the relationship between the target and the environment, these features are used to judge the class of objects, and the high-resolution information is used to provide more fine features, such as gradients, for segmentation targets.

The 3D boundary optimization processing includes: 3D convolution, 3D max-pooling and 3D upward convolution layer. The size of input data is a1, a2 and a3, the number of channels is c, and the size of the filter is f, that is, the dimension of the filter is f*f*f*c, and the number of filters is n, so the final output of 3 dimensional convolution is:

$$(al\text{-}f+1)*(a2\text{-}f+l)*(a3\text{-}f+l)*n,$$

which has an analysis path and a synthesis path. In the analysis path, each layer includes two convolution kernels of 3*3*3, each of which follows an activation function (Relu), and then there is a max-pooling of 2*2*2 on each dimension to merge the two step lengths. In the synthesis path, each layer is composed of 2*2*2 upward convolution, and the step length on each dimension is 2, next, two 3*3*3 convolutions, and then Relu. Shortcut connections from equal resolution layers in the analysis path then provide the basic high-resolution features of the synthetic path. In the last layer, 1*1*1 convolution reduces the number of output channels.

Wherein, the data enhancement processing includes any one of the following: data enhancement based on arbitrary angle rotation, data enhancement based on histogram equalization, data enhancement based on white balance, data enhancement based on mirror operation, data enhancement based on random shearing and data enhancement based on simulating different illumination changes.

It can be seen that the medical imaging apparatus obtains the first medical image data by introducing the BMP data source into a VRDS medical network model, and then introduces the first medical image data into the preset cross blood vessel network model, finally obtains the target medical image data, which improves the convenience of embolism recognition.

S203, performing, by the medical imaging apparatus, 4D medical imaging according to the target medical image data and determines a feature attribute of the embolism according to the imaging results, wherein the feature attribute includes at least one of the following: density, crawling direction, correspondence with a site of cancer focus and edge characteristics.

Wherein, the 4D medical imaging refers to presenting a four-dimensional medical image.

In this possible example, the implementation way of the medical imaging apparatus preforming the 4D medical imaging according to the target medical image data can be: the medical imaging apparatus screens enhanced data with a quality score greater than a preset score from the target medical image data as 4D imaging data, after that performs 4D medical imaging according to the 4D imaging data.

Wherein, the quality score can be comprehensively evaluated from the following dimensions: average gradient, information entropy, visual information fidelity, peak signal-to-noise ratio (PSNR), structural similarity index measurement (SSIM), mean square error (MSE), etc., specific reference can be made to common image quality score algorithms in the image field, which is not described details for brevity.

It can be seen that, in this example, the medical imaging apparatus further performs data screening through the quality score, thus improving the imaging effect.

S204, determining, by the medical imaging apparatus, a type of the embolism according to the feature attribute and outputs the type.

Wherein, the medical imaging apparatus determines the type of embolism according to the feature attribute, which may be that the medical imaging apparatus uses the features of embolism as a query identifier, queries a preset list of the corresponding relationship between the features of embolism and the types of embolism, and obtains the type of embolism corresponding to the features of embolism; or the medical imaging apparatus compares the numerical value corresponding to the features of the embolism with the preset feature value of the embolism to obtain a comparison result, and then determines the type of the embolism according to the comparison result.

Wherein, the outputting of the type by medical imaging apparatus may be displaying the embolism on a display device, or outputting of the type by medical imaging apparatus may be broadcasting the type of embolism by a buzzer, and the medical imaging apparatus can also output the type by other means, which is not particularly limited.

In one possible example, the feature attribute includes density; the determining of the type of embolism according to the feature, including: acquiring a prestored blood vessel embolism density table, wherein the blood vessel embolism density table includes a corresponding relationship between blood vessels at different sites and density intervals of thrombus or cancer thrombus formed in the blood vessels; querying the blood vessel embolism density table to acquire a target density interval to which the density of the embolism of the target site belongs; determining a thrombus or cancer thrombus corresponding to the target density interval as the type of the embolism.

Wherein, the query of the blood vessel embolism density table may take the target site and the embolism density of the target site as a query identifier to query the blood vessel embolism density table. Different sites with the same embolism density may have different embolism types, and different sites with the same embolism density may have the same embolism type.

It can be seen that in this example, the medical imaging apparatus determines the type of embolism by querying the prestored blood vessel embolism density table, which improves the efficiency of recognizing embolism.

In one possible example, the feature attribute includes a crawling direction; the determining of the type of the embolism according to the features including: if it is detected that the crawling direction of the embolism is a reverse blood flow direction, determining that the embolism is a cancer thrombus; if it is detected that the crawling direction of the embolism is a blood flow direction, determining that the embolism is thrombus.

It can be seen that in this example, the medical imaging apparatus determines the type of embolism by recognizing the crawling direction, which improves the convenience of recognizing embolism.

In one possible example, the feature attribute includes correspondence with a site of cancer focus; the determining of the type of the embolism according to the features including: if it is detected that the embolism corresponds to the correspondence with the site of cancer focus, determining that the embolism is a cancer thrombus; if it is detected that the embolism does not correspond to the correspondence with the site of cancer focus, determining that the embolism is thrombus.

Wherein, the site of cancer focus refers to the diseased site where cancer occurs in human body.

It can be seen that in this example, the medical imaging apparatus determines the type of embolism through the correspondence between embolism and the site of cancer focus, which improves the efficiency of recognizing embolism.

In one possible example, the feature attribute includes edge characteristics; the determining of the type of the embolism according to the features attribute, including: if it is detected that the edge characteristic of the embolism is smooth continuous, determining that the embolism is a cancer thrombus; if it is detected that the edge characteristic of the embolism is nonsmooth continuous, determining that the embolism is thrombus.

It can be seen that in this example, the medical imaging apparatus determines the type of embolism through the detecting result on the edge characteristics of embolism, which improves the efficiency of recognizing embolism.

In one possible example, the feature attribute includes density, crawling direction, correspondence with a site of cancer focus and edge characteristics; the determining of the type of the embolism according to the feature attribute including: acquiring a pre-trained embolism recognition model of a target blood vessel at the target site; introducing the density, the crawling direction, the correspondence with the site of cancer focus and the edge characteristics as input data into the embolism recognition model to obtain a first probability that the embolism is a thrombus and a second probability that the embolism is a cancer thrombus; determining the type of the embolism according to the first probability and the second probability.

Wherein, acquiring the embolism recognition model of the target blood vessel of the target site trained in advance may be acquiring the embolism recognition model of the target blood vessel of the target site trained in advance from a network database in a networked state.

Wherein, according to the first probability and the second probability, the type of embolism can be determined as thrombus when the first probability is greater than the second probability, and as cancer thrombus when the first probability is less than the second probability.

In this example, the medical imaging apparatus obtains the type of embolism by introducing the features of embolism density, embolism crawling direction, correspondence between embolism and the site of cancer focus and embolism edge characteristics into the embolism recognition model, which improves the convenience of embolism recognition.

It can be seen that in the embodiment of this application, the medical imaging apparatus first determines a BMP data source according to a plurality of scanned images of a target site of a target user; second, generates target medical image data according to the BMP data source; third, performs 4D medical imaging according to the target medical image data and determines a feature attribute of the embolism according to an imaging result; and finally, determines a type of the embolism according to the feature attribute and outputs the type. Wherein the target site includes an embolism formed on the wall of the target blood vessel, wherein the target medical image data includes a data set of the target blood vessel and a data set of the embolism; the first data in the data set of the target blood vessel and the second data in the data set of the embolism are independent of each other, and the data set of the target blood vessel is a transfer function result of a cubic space between a surface of the target blood vessel and a tissue structure inside the target blood vessel; wherein the feature attribute includes at least one of the following: density, crawling direction, correspondence with a site of cancer focus and edge characteristics. It can be seen that the medical imaging apparatus in this application can accurately determine the type of embolism from the imaging results of 4D medical imaging, and improve the accuracy and efficiency of embolism recognition.

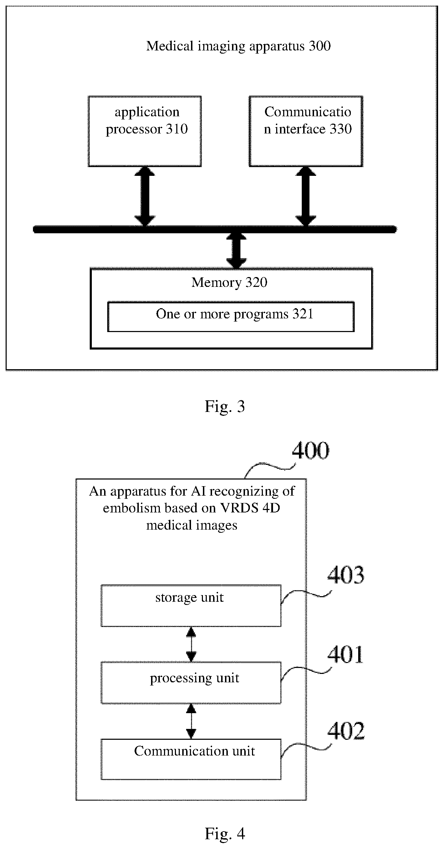

Consistent with the embodiments shown in Fig. 2, referring to Fig. 3, Fig. 3 is a schematic structural diagram of a medical imaging apparatus 300 provided by an embodiment of this application, as shown in the figure, the medical imaging apparatus 300 includes a processor 310, a memory 320, a communication interface 330 and one or more programs 321, wherein the one or more programs 321 are stored in the memory 320 and configured to be executed by the processor 310, and the one or more programs 321 include instructions for executing the following steps: determining a BMP data source according to a plurality of scanned images of a target site of a target user, wherein the target site includes an embolism formed on the wall of the target blood vessel; generating a target medical image data according to the BMP data source, wherein the target medical image data includes a data set of the target blood vessel and a data set the embolism; the first data in the data set of the target blood vessel and the second data in the data set of the embolism are independent of each other, and the data set of the target blood vessel is a transfer function result of a cubic space between a surface of the target blood vessel and a tissue structure inside the target blood vessel; performing 4D medical imaging according to the target medical image data and determining a feature attribute of the embolism according to an imaging result, wherein the feature attribute includes at least one of the following: density, crawling direction, correspondence with a site of cancer focus and edge characteristics; and determining a type of the embolism according to the feature and outputting the type.

It can be seen that in the embodiment of this application, the medical imaging apparatus first determines a BMP data source according to a plurality of scanned images of a target site of a target user; second, generates target medical image data according to the BMP data source; third, performs 4D medical imaging according to the target medical image data and determines a feature attribute of the embolism according to an imaging result; and finally, determines a type of the embolism according to the features and outputs the type. Wherein the target site includes an embolism formed on the wall of the target blood vessel, wherein the target medical image data includes a data set of the target blood vessel and a data set of the embolism; the first data in the data set of the target blood vessel and the second data in the data set of the embolism are independent of each other, and the data set of the target blood vessel is a transfer function result of a cubic space between a surface of the target blood vessel and a tissue structure inside the target blood vessel; wherein the feature attribute includes at least one of the following: density, crawling direction, correspondence with a site of cancer focus and edge characteristics. It can be seen that the medical imaging apparatus in this application can accurately determine the type of embolism from the imaging results of 4D medical imaging, and improve the accuracy and efficiency of embolism recognition.

In one possible example, the feature attribute includes density. In the aspect of the determining of the type of embolism according to the feature, the instructions in the program are specifically configured to perform the following operation: acquiring a prestored blood vessel embolism density table, wherein the blood vessel embolism density table includes a corresponding relationship between blood vessels at different sites and density intervals of thrombus or cancer thrombus formed in the blood vessels; querying the blood vessel embolism density table to acquire a target density interval to which the density of the embolism of the target site belongs; determining a thrombus or cancer thrombus corresponding to the target density interval as the type of the embolism.

In one possible example, the feature attribute includes a crawling direction. In the aspect of the determining of the type of the embolism according to the features, the instructions in the program are specifically configured to perform the following operation: if it is detected that the crawling direction of the embolism is a reverse blood flow direction, determining that the embolism is a cancer thrombus; if it is detected that the crawling direction of the embolism is a blood flow direction, determining that embolism is thrombus.

In one possible example, the feature attribute includes correspondence with a site of cancer focus. In the aspect of the determining of the type of the embolism according to the features, the instructions in the program are specifically configured to perform the following operation: the program further includes instructions configured to perform the following operation: if it is detected that the embolism corresponds to the correspondence with the site of cancer focus, determining that the embolism is a cancer thrombus; if it is detected that the embolism is not correspond to the correspondence with the site of cancer focus, determining that the embolism is thrombus.

In one possible example, the feature attribute includes edge characteristics. In the aspect of the determining of the type of the embolism according to the features, the instructions in the program are specifically configured to perform the following operation: if it is detected that the edge characteristic of the embolism is smooth continuous, determining that the embolism is a cancer thrombus; if it is detected that the edge characteristic of the embolism is nonsmooth continuous, determining that the embolism is thrombus.

In one possible example, the feature attribute includes density, crawling direction, correspondence with a site of cancer focus and edge characteristics. In the aspect of the determining of the type of the embolism according to the features, the instructions in the program are specifically configured to perform the following operation: acquiring a pre-trained embolism recognition model of a target blood vessel at the target site; introducing the density, the crawling direction, the correspondence with the site of cancer focus and the edge characteristics as input data into the embolism recognition model to obtain a first probability that the embolism is a thrombus and a second probability that the embolism is a cancer thrombus; determining the type of the embolism according to the first probability and the second probability.

In one possible example, in the aspect of the generating of the target medical image data according to the BMP data source, the instructions in the program are specifically configured to perform the following operation: introducing the BMP data source into a preset VRDS medical network model to obtain first medical image data, wherein the first medical image data includes an raw data set of the target blood vessel; and the raw data set of the target blood vessel includes fusion data of the target blood vessel and the embolism; introducing the first medical image data into a preset cross blood vessel network model and performing spatial segmentation processing on the fusion data through the cross blood vessel network model to obtain a data set of the target blood vessel and a data set of the embolism; synthesizing the data set of the target blood vessel and the data set of the embolism to obtain the target medical image data.

The above mainly introduces the solution of the embodiment of this application from the perspective of the execution process on the method side. It can be understood that in order to achieve the above functions, the medical imaging apparatus includes corresponding hardware structures and/or software modules for performing various functions. It should be easy for the skilled person in the art aware that, in combination with the units and algorithmic steps of the examples described in the embodiments provided herein, this application can be implemented in the form of hardware or a combination of hardware and computer software. Whether the functions are performed by hardware or computer software driving hardware depends on particular applications and design constraint conditions of the technical solutions. A person skilled in the art may use different methods to implement the described functions for each particular application, but it should not be considered that the implementation goes beyond the scope of this application.

The embodiment of this application can divide the medical imaging apparatus into functional units according to the above method example, for example, individual functional unit can be divided corresponding to individual function, or two or more functions can be integrated into one processing unit. The integrated units can be implemented in the form of hardware, and can also be implemented in the form of a software functional unit. It should be noted that the division of units in the embodiment of this application is schematic, which is only a logical function division, and there may be another division mode in actual implementation.

Fig. 4 is a block diagram of functional units composition of an apparatus 400 for AI recognizing of embolism based on VRDS AI 4D medical images involved in the embodiment of this application. The apparatus 400 for AI recognizing of embolism based on VRDS AI 4D medical images is applied to a medical imaging apparatus, the apparatus 400 for AI recognizing of embolism based on VRDS AI 4D medical image includes a processing unit 401 and a communication unit 402, wherein,
the processing unit 401 is configured to: determine a BMP data source according to a plurality of scanned images of a target site of a target user, wherein the target site includes an embolism formed on the wall of the target blood vessel; generate target medical image data according to the BMP data source, wherein the target medical image data includes a data set of the target blood vessel and a data set the embolism; the first data in the data set of the target blood vessel and the second data in the data set of the embolism are independent of each other, and the data set of the target blood vessel is a transfer function result of a cubic space between a surface of the target blood vessel and a tissue structure inside the target blood vessel; perform 4D medical imaging according to the target medical image data and determine a feature attribute of the embolism according to an imaging result, wherein the feature attribute includes at least one of the following: density, crawling direction, correspondence with a site of cancer focus and edge characteristics; and determine a type of the embolism according to the feature and output the type through the communication unit 402.

The apparatus 400 for AI recognizing of embolism based on VRDS AI 4D includes a storage unit 403 for storing program codes and data of electronic device. The processing unit 401 can be a processor, the communication unit 402 can be a transceiver, and the storage unit 403 can be a memory.

It can be seen that in the embodiment of this application, the medical imaging apparatus first determines a BMP data source according to a plurality of scanned images of a target site of a target user; second, generates target medical image data according to the BMP data source; third, performs 4D medical imaging according to the target medical image data and determines a feature attribute of the embolism according to an imaging result; and finally, determines a type of the embolism according to the features and outputs the type. Wherein the target site includes an embolism formed on the wall of the target blood vessel, wherein the target medical image data includes a data set of the target blood vessel and a data set of the embolism; the first data in the data set of the target blood vessel and the second data in the data set of the embolism are independent of each other, and the data set of the target blood vessel is a transfer function result of a cubic space between a surface of the target blood vessel and a tissue structure inside the target blood vessel, wherein the feature attribute includes at least one of the following: density, crawling direction, correspondence with a site of cancer focus and edge characteristics. It can be seen that the medical imaging apparatus in this application can accurately determine the type of embolism from the imaging results of 4D medical imaging, and improve the accuracy and efficiency of embolism recognition.

In one possible example, the feature attribute includes density. In the aspect of the determining of the type of embolism according to the feature, the processing unit 401 is specifically configured to: acquire a prestored blood vessel embolism density table, wherein the blood vessel embolism density table includes a corresponding relationship between blood vessels at different sites and density intervals of thrombus or cancer thrombus formed in the blood vessels; query the blood vessel embolism density table to acquire a target density interval to which the density of the embolism of the target site belongs; determine a thrombus or cancer thrombus corresponding to the target density interval as the type of the embolism.

In one possible example, the feature attribute includes a crawling direction. In the aspect of the determining of the type of the embolism according to the features, the processing unit 401 is specifically configured to: if it is detected that the crawling direction of the embolism is a reverse blood flow direction, determine that the embolism is a cancer thrombus; if it is detected that the crawling direction of the embolism is a blood flow direction, determine that the embolism is thrombus.

In one possible example, the feature attribute includes correspondence with a site of cancer focus. In the aspect of the determining of the type of the embolism according to the features, the processing unit 401 is specifically configured to: if it is detected that the embolism corresponds to the correspondence with the site of cancer focus, determine that the embolism is a cancer thrombus; if it is detected that the embolism is not correspond to the correspondence with the site of cancer focus, determine that the embolism is thrombus.

In one possible example, the feature attribute includes edge characteristics. In the aspect of the determining of the type of the embolism according to the features, the processing unit 401 is specifically configured to: if it is detected that the edge characteristic of the embolism is smooth continuous, determine that the embolism is a cancer thrombus; if detecting that the edge characteristic of the embolism is nonsmooth continuous, determine that the embolism is thrombus.

In one possible example, the feature attribute includes density, crawling direction, correspondence with a site of cancer focus and edge characteristics. In the aspect of the determining of the type of the embolism according to the features, the processing unit 401 is specifically configured to: acquire a pre-trained embolism recognition model of a target blood vessel at the target site; introduce the density, the crawling direction, the correspondence with the site of cancer focus and the edge characteristics as input data into the embolism recognition model to obtain a first probability that the embolism is a thrombus and a second probability that the embolism is a cancer thrombus; determine the type of the embolism according to the first probability and the second probability.

In one possible example, in the aspect of the generating of the target medical image data according to the BMP data source, the processing unit 401 is specifically configured to: introduce the BMP data source into a preset VRDS medical network model to obtain first medical image data, wherein the first medical image data includes an raw data set of the target blood vessel, and the raw data set of the target blood vessel includes fusion data of the target blood vessel and the embolism; introduce the first medical image data into a preset cross blood vessel network model and perform spatial segmentation processing on the fusion data through the cross blood vessel network model to obtain a data set of the target blood vessel and a data set of the embolism; synthesize the data set of the target blood vessel and the data set of the embolism to obtain the target medical image data.

Embodiments of this application further provide a computer storage medium, wherein the computer storage medium stores a computer program for electronic data exchange, the computer program causes a computer to execute part or all of the steps of any method as recorded in the above method embodiment, and the computer includes a medical imaging apparatus.

Embodiments of this application further provide a computer program product, the computer program product includes a non-transitory computer-readable storage medium in which a computer program is stored, the computer program is operable to cause a computer to execute part or all of the steps of any method as recorded in the above method embodiments. The computer program product can be a software installation package, and the computer includes a medical imaging apparatus.

It should be noted that, for brevity of description, the foregoing method embodiments are described as a series of movement combinations. However, the skilled person in the art should learn that this application is not limited by the movement sequence, because according to this application, some steps can be performed in other order or simultaneously. second, it also should be known by the skilled person in the art that the embodiments described in the specification are preferred embodiments and the involved actions and modules are not the must of the present application necessarily.

In the above embodiments, the descriptions of individual embodiment have their own emphasis, for those parts that are not detailed in one embodiment, please refer to the relevant descriptions of other embodiments.

In the several embodiments provided in this application, it should be understood that the disclosed apparatus may be implemented in other manners. For example, the described apparatus embodiment is merely an example. For example, the above unit division is merely logical function division and may be other division in actual implementation. For example, a plurality of units or components may be combined or integrated into another system, or some features may be ignored or not performed. In addition, the displayed or discussed mutual couplings or direct couplings or communication connections may be implemented by using some interfaces. The indirect couplings or communication connections between the apparatuses or units may be implemented in electronic, or other forms.

The above units described as separate parts may or may not be physically separate, and parts displayed as units may or may not be physical units, may be located in one position, or may be distributed on a plurality of network units. It can select some or all of units to achieve the objective of the solution of the present embodiment based on actual requirements.

In addition, functional units in the embodiments of this application may be integrated into one processing unit, or each of the units may exist alone physically, or two or more units are integrated into one unit. The integrated units can be implemented in the form of hardware, and can also be implemented in the form of a software functional unit.

When the above integrated units are implemented in the form of a software functional unit and sold or used as an independent product, the functions may be stored in a computer readable memory. Based on such an understanding, the technical solutions of this application essentially, or the part contributing to the prior art, all or some of the technical solutions may be implemented in a form of a software product. The computer software product is stored in a memory, and includes several instructions for instructing a computer device (which may be a personal computer, a server, or a network device) to perform all or some of the steps of the above methods described in the embodiments of this application. The foregoing memory includes: any medium that can store program code, such as a USB flash disk, a read-only memory (ROM), a random access memory (RAM) removable hard disk, a magnetic disk, or an optical disc.

The ordinary skill person in the art may understand that all or some of the steps in various methods of the above embodiments can be completed by instructing related hardware through programs, which can be stored in a computer readable memory, which may include: flash disks, Read-Only Memory (ROM) random access memory (RAM) disks or optical disks, etc.

The embodiments of this application are described in detail above, and the principles and implementation of this application are explained by specific examples. The above embodiments are only used to help understand the method and its core ideas of this application; at the same time, according to the idea of this application, there will be some changes in the specific implementation and application scope for a person of skill in the art, to sum up, the contents of this specification should not be construed as limitations of this application.

**Claims**

1. A method for AI recognizing of embolism based on Virtual Reality Doctor system (VRDS) 4D medical images, **characterized in that** the method is applied to a medical imaging apparatus; and the method comprises:

   determining a bitmap (BMP) data source according to a plurality of scanned images of a target site of a target user, wherein the target site comprises an embolism formed on a wall of a target blood vessel;
   generating a target medical image data according to the BMP data source, wherein the target medical image data comprises a data set of the target blood vessel and a data set the embolism; first data in the data set of the target blood vessel and second data in the data set of the embolism are independent of each other, and the data set of the target blood vessel is a transfer function result of a cubic space between a surface of the target blood vessel and a tissue structure inside the target blood vessel;
   performing 4D medical imaging according to the target medical image data and determining a feature attribute of the embolism according to the imaging results, wherein the feature attribute comprises at least one of the following: density, crawling direction, correspondence with a site of cancer focus and edge characteristics;
   determining a type of the embolism according to the feature attribute and outputting the type.

2. The method according to claim 1, **characterized in that** the feature attribute comprises density; the determining of the type of the embolism according to the feature attribute comprises:

   acquiring a prestored blood vessel embolism density table, wherein the blood vessel embolism density table comprises a corresponding relationship between blood vessels at different sites and density intervals of thrombus or cancer thrombus formed in the blood vessels;
   querying the blood vessel embolism density table to acquire a target density interval to which the density of the embolism of the target site belongs;
determining a thrombus or cancer thrombus corresponding to the target density interval as the type of the embolism.

3. The method according to claim 1, **characterized in that** the feature attribute includes a crawling direction; the determining of the type of the embolism according to the feature attribute includes:
   if it is detected that the crawling direction of the embolism is a reverse blood flow direction, determining that the embolism is a cancer thrombus; if it is detected that the crawling direction of the embolism is a blood flow direction, determining that the embolism is thrombus.

4. The method according to claim 1, **characterized in that** the feature attribute includes correspondence with a site of cancer focus; the determining of the type of the embolism according to the feature attribute comprises:

   if it is detected that the embolism corresponds to the site of cancer focus, determining that the embolism is a cancer thrombus;
   if it is detected that the embolism does not correspond to the site of cancer focus, determining that the embolism is thrombus.

5. The method according to claim 1, **characterized in that** the feature attribute comprises edge characteristic; the determining of the type of the embolism according to the feature attribute comprises:

   if it is detected that the edge characteristic of the embolism is smooth continuous, determining that the embolism is a cancer thrombus;
   if it is detected that the edge characteristic of the embolism is nonsmooth continuous, determining that the embolism is thrombus.

6. The method according to claim 1, **characterized in that** the feature attribute comprises density, crawling direction, correspondence with a site of cancer focus and edge characteristics; the determining of the type of the embolism according to the feature attribute comprises:

   acquiring a pre-trained embolism recognition model of the target blood vessel at the target site;
   introducing the density, the crawling direction, the correspondence with the site of cancer focus and the edge characteristics as input data into the embolism recognition model to obtain a first probability that the embolism is a thrombus and a second probability that the embolism is a cancer thrombus;

determining the type of the embolism according to the first probability and the second probability.

7. The method according to any one of claims 1-6, **characterized in that** the generating the target medical image data according to the BMP data source comprises:

introducing the BMP data source into a preset VRDS medical network model to obtain a first medical image data, wherein the first medical image data comprises an raw data set of the target blood vessel, and the raw data set of the target blood vessel comprises fusion data of the target blood vessel and the embolism; introducing the first medical image data into a preset cross blood vessel network model; and performing spatial segmentation processing on the fusion data through the cross blood vessel network model to obtain a data set of the target blood vessel and a data set of the embolism; synthesizing the data set of the target blood vessel and the data set of the embolism to obtain the target medical image data.

8. The method according to claim 7, **characterized in that** the synthesizing of the data set of the target blood vessel and the data set of the embolism to obtain the target medical image data comprises: executing a second preset processing on the data set of the target blood vessel and the data set of the embolism to obtain the target medical image data; the second preset processing comprises at least one of the following operations: 2D boundary optimization processing, 3D boundary optimization processing and data enhancement processing.

9. The method according to claim 1, **characterized in that** the outputting of the type comprises: displaying the type of the embolism on a display device.

10. An apparatus for AI recognizing of embolism based on VRDS 4D medical images, **characterized in that** the apparatus is applied to a medical imaging apparatus; the AI recognition apparatus of embolism based on VRDS AI 4D medical image comprises a processing unit and a communication unit, wherein the processing unit is configured to: determine a bitmap (BMP) data source according to a plurality of scanned images of a target site of a target user, wherein the target site comprises an embolism formed on a wall of a target blood vessel; generate a target medical image data according to the BMP data source, wherein the target medical image data comprises a data set of the target blood vessel and a data set the embolism; a first data in the data set of the target blood vessel and a second data in the data set of the embolism are independent of each other, and the data set of the target blood vessel is a transfer function result of a cubic space between a surface of the target blood vessel and a tissue structure inside the target blood vessel; perform 4D medical imaging according to the target medical image data and determine a feature attribute of the embolism according to an imaging result, wherein the feature attribute comprises at least one of the following: density, crawling direction, correspondence with a site of cancer focus and edge characteristics; and determine a type of the embolism according to the feature attribute and output the type through the communication unit.

11. The apparatus according to claim 10, **characterized in that** the feature attribute comprises density; in the aspect of the determining of the type of embolism according to the feature attribute, the processing unit is specifically configured to: acquire a prestored blood vessel embolism density table, wherein the blood vessel embolism density table comprises a corresponding relationship between blood vessels at different sites and density intervals of thrombus or cancer thrombus formed in the blood vessels; query the blood vessel embolism density table to acquire a target density interval to which the density of the embolism of the target site belongs; determine a thrombus or cancer thrombus corresponding to the target density interval as the type of the embolism.

12. The apparatus according to claim 10, **characterized in that** the feature attribute comprises a crawling direction; in the aspect of the determining of the type of the embolism according to the features, the processing unit is specifically configured to: if it is detected that the crawling direction of the embolism is a reverse blood flow direction, determine that the embolism is a cancer thrombus; if detecting that the crawling direction of the embolism is a blood flow direction, determine that the embolism is thrombus.

13. The apparatus according to claim 10, **characterized in that** the feature attribute comprises correspondence with a site of cancer focus; in the aspect of the determining of the type of the embolism according to the features, the processing unit is specifically configured to: if it is detected that the embolism corresponds to the site of cancer focus, determine that the embolism is a cancer thrombus; if it is detected that the embolism does not correspond to the site of cancer focus, determine that the embolism is thrombus.

14. The apparatus according to claim 10, **characterized in that** the feature attribute comprises edge characteristics; in the aspect of the determining of the type of the embolism according to the features, the processing unit is specifically configured to: if it is detected that the edge characteristic of the embolism is smooth continuous, determine that the embolism is a cancer thrombus; if it is detected that the edge characteristic of the embolism is nonsmooth continuous, determine that the embolism is thrombus.

15. The apparatus according to claim 10, **characterized in that** the feature attribute comprises density, crawling direction, correspondence with the site of cancer focus and edge characteristics; in the aspect of the determining of the type of the embolism according to the feature attribute, the processing unit is specifically configured to: acquire a pre-trained embolism recognition model of the target blood vessel at the target site; introduce the density, the crawling direction, the correspondence with the site of cancer focus and the edge characteristics as input data into the embolism recognition model to obtain a first probability that the embolism is a thrombus and a second probability that the embolism is a cancer thrombus; determine the type of the embolism according to the first probability and the second probability.

16. The apparatus according to any one of claims 10-15, **characterized in that** in the aspect of the generating of the target medical image data according to the BMP data source, the processing unit is specifically configured to: introduce the BMP data source into a preset VRDS medical network model to obtain a first medical image data, wherein the first medical image data comprises an raw data set of the target blood vessel, and the raw data set of the target blood vessel comprises fusion data of the target blood vessel and the embolism; introduce the first medical image data into a preset cross blood vessel network model and perform spatial segmentation processing on the fusion data through the cross blood vessel network model to obtain a data set of the target blood vessel and a data set of the embolism; synthesize the data set of the target blood vessel and the data set of the embolism to obtain the target medical image data.

17. The apparatus according to claim 16, **characterized in that** in the aspect of the synthesizing of the data set of the target blood vessel and the data set of the embolism to obtain the target medical image data, the processing unit is specifically configured to: execute a second preset processing on the data set of the target blood vessel and the data set of the embolism to obtain the target medical image data, wherein the second preset processing comprises at least one of the following operations: 2D boundary optimization processing, 3D boundary optimization processing and data enhancement processing.

18. The apparatus according to claim 10, **characterized in that** in the aspect of the outputting of the type, the output unit is specifically configured to: display the type of the embolism on a display device.

19. A medical imaging apparatus, **characterized in that** the apparatus comprises a processor, a memory, a communication interface, and one or more programs; the one or more programs are stored in the memory and configured to be executed by the processor, and the programs comprise instructions for executing the steps in the method according to any one of claims 1-9.

20. A computer readable storage medium, **characterized in that** the computer readable storage medium stores a computer program for electronic data exchange, wherein the computer program causes a computer to execute the method according to any one of claims 1-9.

100

120

111    112

110

Fig. 1

Determining, by the medical imaging apparatus, a bitmap (BMP) data source according to a plurality of scanned images of a target site of a target user    S201

Generating, by the medical imaging apparatus, target medical image data according to the BMP data source    S202

Performing, by the medical imaging apparatus, a 4D medical imaging according to the target medical image data, and determining a feature attribute of the embolism according to the imaging results    S203

Determining, by the medical imaging apparatus, a type of the embolism according to the features and outputting the type    S204

Fig. 2

Medical imaging apparatus 300

application processor 310

Communication interface 330

Memory 320

One or more programs 321

Fig. 3

400

An apparatus for AI recognizing of embolism based on VRDS 4D medical images

storage unit 403

processing unit 401

Communication unit 402

Fig. 4

## INTERNATIONAL SEARCH REPORT

| International application No. |
|---|
| **PCT/CN2019/101159** |

**A. CLASSIFICATION OF SUBJECT MATTER**

A61B 6/03(2006.01)i; G06T 7/00(2017.01)i

According to International Patent Classification (IPC) or to both national classification and IPC

**B. FIELDS SEARCHED**

Minimum documentation searched (classification system followed by classification symbols)

A61B,G06T

Documentation searched other than minimum documentation to the extent that such documents are included in the fields searched

Electronic data base consulted during the international search (name of data base and, where practicable, search terms used)

CNPAT, CNKI, WPI, EPODOC: 影像, 类, "4D", 栓塞, 种类, 表面, 类别, 结构, 类型, 血栓, 图像, 血管, 数据, 单独, 分开, 独立, 分类, AI, video, kind, type, class, vategory, surface, façade, thrombus, blood, vessel, haemal, wall, absolute

**C. DOCUMENTS CONSIDERED TO BE RELEVANT**

| Category* | Citation of document, with indication, where appropriate, of the relevant passages | Relevant to claim No. |
|---|---|---|
| A | CN 109259784 A (SHANGHAI DR BRAIN TECH CO., LTD.) 25 January 2019 (2019-01-25) description, paragraphs [0052]-[0073] | 1-20 |
| A | CN 104376549 A (NORTH CHINA ELECTRIC POWER UNIVERSITY (BAODING)) 25 February 2015 (2015-02-25) entire document | 1-20 |
| A | CN 105279759 A (ZHEJIANG UNIVERSITY OF TECHNOLOGY) 27 January 2016 (2016-01-27) entire document | 1-20 |
| A | CN 107993221 A (UNIVERSITY OF JI'NAN) 04 May 2018 (2018-05-04) entire document | 1-20 |
| A | US 2011243403 A1 (FUJIFILM CORP.) 06 October 2011 (2011-10-06) entire document | 1-20 |

☐ Further documents are listed in the continuation of Box C.　　☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **18 October 2019** | **20 November 2019** |

| Name and mailing address of the ISA/CN | Authorized officer |
|---|---|
| **China National Intellectual Property Administration (ISA/ CN)** **No. 6, Xitucheng Road, Jimenqiao Haidian District, Beijing 100088** **China** | |
| Facsimile No. **(86-10)62019451** | Telephone No. |

Form PCT/ISA/210 (second sheet) (January 2015)

EP 3 928 706 A1

INTERNATIONAL SEARCH REPORT
Information on patent family members

International application No.

**PCT/CN2019/101159**

| Patent document cited in search report | | | Publication date (day/month/year) | Patent family member(s) | | | Publication date (day/month/year) |
|---|---|---|---|---|---|---|---|
| CN | 109259784 | A | 25 January 2019 | None | | | |
| CN | 104376549 | A | 25 February 2015 | None | | | |
| CN | 105279759 | A | 27 January 2016 | None | | | |
| CN | 107993221 | A | 04 May 2018 | None | | | |
| US | 2011243403 | A1 | 06 October 2011 | JP | 2011212313 | A | 27 October 2011 |
| | | | | EP | 2375379 | A2 | 12 October 2011 |
| | | | | CN | 102208105 | A | 05 October 2011 |

Form PCT/ISA/210 (patent family annex) (January 2015)

17